# FASCICULE DE BREVET EUROPEEN

(11) **EP 1 958 205 B1**
(45) Date de publication et mention de la délivrance du brevet: **16.01.2013**
(21) Numéro de dépôt: 06818815.0
(22) Date de dépôt: 24.11.2006
(51) Int. Cl.: G11C 13/00, G11C 13/02, C12Q 1/00, C12Q 1/68, G01N 33/552, G01N 33/543, G01N 33/553, B82Y 10/00

(54) **DISPOSITIF NANOSTRUCTURE**
NANOSTRUKTURIERTE ANORDNUNG
NANOSTRUCTURED DEVICE

(30) Priorité: 25.11.2005 FR 0511986
(43) Date de publication de la demande: 20.08.2008
(73) Titulaire: Commissariat à l'Énergie Atomique et aux Énergies Alternatives, 75015 Paris (FR)
(72) Inventeur: MUR, Pierre, F-38920 Crolles (FR); OILLIC, Cécile, F-44160 Pont-Chateau (FR)
(74) Mandataire: Stolmár, Matthias
(86) Numéro de dépôt international: PCT/EP2006/011300
(87) Numéro de publication internationale: WO 2007/059996

(56) Documents cités:
- EP-A- 1 376 606
- WO-A-03/052835
- WO-A-2005/105308
- WO-A2-2004/099068
- US-A- 6 159 620
- BARON T ET AL: "GROWTH LOW PRESSURE CHEMICAL VAPOR DEPOSITION OF SILICON QUANTUM DOTS ON INSULATOR FOR NANOELECTRONICS DEVICES" SEMICONDUCTOR QUANTUM DOTS. SAN FRANCISCO, CA, APRIL 5 - 8, 1999, MATERIALS RESEARCH SOCIETY SYMPOSIUM PROCEEDINGS. VOL. 571. (MRS), WARRENDALE, PA : MRS, US, vol. VOL. 571, 5 avril 1999 (1999-04-05), pages 37-42, XP000922450 ISBN: 1-55899-478-5
- LISA R. HILLARD ET AL: "Immobilization of oligonucleotides onto silica nanoparticles for DNA hybridization studies" ANALYTICA CHINICA ACTA, vol. 470, 2002, pages 51-56, XP002403577
- BAWENDI M G: "NANOCRYSTALITES: BUILDING BLOCKS FOR QUANTUM DOT HETEROSTRUCTURES" SOLID STATE COMMUNICATIONS, OXFORD, GB, vol. 107, no. 11, 1998, page 709, XP000937488 ISSN: 0038-1098

## Description

La présente invention a pour objet un dispositif nanostructuré avec une pluralité des sites nanostructurés tridimensionnels pour le greffage des espèces chimiques. Le dispositif selon l'invention est utilisable pour la fabrication d'une biopuce et une mémoire électronique. La présente invention a également pour objet un procédé pour la formation d'un tel dispositif monostructuré.

Conférer des propriétés de surfaces bien particulières à la surface d'un microsystème (microstructure) est une problématique importante dans le domaine des micro et nanotechnologies. Il est encore plus important de pouvoir disposer de ces différentes propriétés sur un même support.

La fonctionnalisation de surface est souvent assurée par la réaction d'un silane organique sur une couche d'oxyde par exemple SiO₂. On notera de nombreux exemples. L'application de la couche organique peut être réalisée par exemple par trempage en phase liquide (cf. "Nanoliter liquid metering in microchannels using hydrophobic patterns" Anal. Chem. 2000, 72, 4100-4109), en phase gazeuse, par embossing (cf. "Chemical nano-patterning using hot embossing lithography", Microelectronics Engineering 2002, 61-62, 423-428), par dépôt, par spin coating (cf. "Automatic transportation of a droplet on a wettability gradient surface", 7th International Conference on miniaturized Chemical and Biochemical analysis systems, October 5-9 2003, Sqaw Valley, CA (USA)) etc..

L'immobilisation des molécules organiques sur les supports nécessite la création d'une interface entre le substrat inorganique (verre, oxyde de silicium...) et les molécules d'intérêts grâce à la fonctionnalisation des surfaces. Différents couples substrat/molécules d'intérêts possédant des fonctions chimiques appropriées peuvent être utilisés afin d'introduire une liaison mettant en jeu différentes interactions.

Actuellement, dans le domaine de la biologie, deux principales techniques d'immobilisation de molécules biologiques sont couramment utilisées pour la préparation de biopuces à acides nucléiques. Selon le type d'application recherché, les sondes sont immobilisées de manière covalente ou non par une des extrémités sur un support solide. La mise en oeuvre des blopuces est effectuée soit par des technologies dites « on chip » où les sondes oligonucléotides sont synthétisées in situ sur le support ou par des technologies dites « off chip » où les sondes sont préalablement synthétisées, purifiées et contrôlées avant d'être greffées sur le substrat grâce à un robot de dépôt.

A l'heure actuelle, la majeure partie des biopuces commercialisées est réalisée sur un support en verre, fonctionnalisées par divers groupements fonctionnels. Bien que la qualité et la fiabilité de la plupart des biopuces ne soient plus à démontrer, la chimie de fonctionnalisation utilisée sur les surfaces planes génère un seul groupement fonctionnel par molécule que ce soit de la part du support ou de l'acide nucléique. Or, l'augmentation des sites actifs sur lesquels les sondes peuvent se lier permet d'augmenter la capacité de sondes Immobilisées sur le support d'où une meilleure sensibilité de la réponse en terme d'hybridation.

Différentes stratégies ont été envisagées pour améliorer la sensibilité de la détection des événements biologiques. Une première voie proposée est d'accroître la densité de réaction d'hybridation à la surface de la blopuce. Ceci peut être réalisé par l'augmentation soit de la surface spécifique disponible en utilisant des supports poreux soit de la densité de sondes greffées en utilisant des surfaces tridimensionnelles (Yap et. al, Langmuir 2005, vol. 21, No. 12).

Plusieurs équipes ont travaillé sur l'élaboration d'une structure pseudo tridimensionnelle basée sur un système d'accroche appelé « dendrimères ». Les études successives ont permis de limiter le nombre d'étapes pour la réalisation de ce support; néanmoins, la fabrication du support nécessite malgré tout de réaliser indépendamment le support et les éléments à reporter, ce qui implique un coût important et un grand nombre d'étapes (Le Berre, Nud. Acids Res. 2003, 31/No16 e 88).

US 6,159,620 divulgue un dispositif nanostructuré comprenant un substrat, une couche intermédiaire, une zone comportant une pluralité des sites nanostructurés sous forme des nanocristaux en argent fonctionnalisés avec des molécules organiques ayant des fonctionnalités spécifiques qui sont realisés directement sur une electrode sous forme d'un film.

Baron et al. (Mat.Res. Soc. Symp. Proc. Vol.571, p. 37-42) ont présenté une etude sur la nucleation et la croissance des nanocristallites de Si sur des substrats comme le SiO₂, SiOₖN_{y} et Si₃N₄. en utilisant un réacteur du type LPCVD (Low iPressure Chemical vapor Deposition) à basse temperature. La densité et la taille des nanocristallites de Si est influencé par la nature chimique de la surface et la présence des liaisons SiO.

D'autres auteurs ont utilisé des nano particules modifiées et Immobilisées sur des surfaces planes comme autre vole pour réduire l'encombrement stérique à la surface et augmenter la densité de sondes pour l'hybridation. Les nano particules comme les supports récepteurs de ces éléments peuvent être de natures très diverses (métallique, latex de polystyrène, silice sur du verre, silicium, polystyrène-polybutadiène...). Cependant, cette méthode, comme le procédé décrit précédemment, nécessite le report des éléments sur une surface préparée à part, d'où une réalisation de la biopuce en plusieurs étapes et un coût élevé.

Dans le cas des mémoires à stockage de charges, une des solutions utilisées pour augmenter la capacité des plans mémoires Flash, sans avoir à diminuer la taille de cellule élémentaire, est d'utiliser la programmation multi-niveaux. On parle alors de mémoires à multi-niveaux (ou MLC pour Multi-Level-Cell). La programmation multi-niveaux permet de coder plusieurs bits dans la même cellule. Elle consiste à quantifier la charge stockée sur la grille flottante en 2" (n>1) états afin d'avoir la possibilité de coder « n » bits plutôt qu'un seul. Pour cela, on assigne une séquence de bits à une plage de tension spécifique. Par exemple, chaque cellule peut donc prendre les états 00, 01, 10 ou 11, correspondant à autant de niveaux de tension de seuil, qui eux-mêmes dépendent de la charge stockée dans la grille flottante du transistor. Ce concept a été présenté en 1995 par Intel qui l'a ensuite utilisé dans une famille de produits nommée « Strataflash ». La mise en oeuvre de la programmation multi-niveaux n'est cependant pas facile. La difficulté est de contrôler avec précision la charge stockée sur chacun des états de la cellule et de les discriminer avec précision (lecture précise des différents niveaux de tension de seuil).

Les capacités des mémoires flash n'ont pas cessé d'augmenter avec la réduction continuelle des cellules élémentaires liée à la miniaturisation des dispositifs. Cette course à la densité d'intégration ainsi qu'à la diminution des temps d'opérations a permis de faire augmenter la densité des mémoires de 64Mbits en 1997 à 512Mbits actuellement pour les Flash NOR et 2Gbits pour les NAND. Des réalisations de prototypes de Flash NAND 8Gbits en technologie 63 nm en utilisant la technique MLC (Multi-Level-Cell) ont été également présentées par Samsung fin 2004.

Cependant, plusieurs obstacles technologiques commencent à s'opposer à la poursuite de cette miniaturisation. La réduction des dimensions des mémoires s'accompagne de la réduction des épaisseurs des diélectriques, en particulier de l'oxyde de grille. L'épaisseur d'oxyde tunnel sera en 2007 de 8-9 nm pour les NOR et de 6-7 nm pour les NAND. La réduction de l'épaisseur de cet oxyde au dessous de 8 nm, donne lieu à une augmentation des courants de fuite à travers ce dernier, par effet tunnel direct ou par des défauts dans cet oxyde, la rétention en est alors affectée. La diminution des tensions de fonctionnement pour réduire la consommation d'énergie afin d'atteindre les faibles tensions d'utilisation (∼1 à 2 Volts) des transistors CMOS (Complementary MOS) de logique est une autre difficulté à résoudre.

Pour pallier ces difficultés de nouvelles architectures et de nouveaux matériaux sont aujourd'hui étudiés pour les mémoires Flash.

Le remplacement de la grille flottante en silicium polycristallin par des sites de piégeage discrets a été proposé. Un des intérêts de ce type de pièges est l'isolement électrique entre sites de piégeage.

La présence d'un défaut dans l'oxyde tunnel n'affectera que la charge située au dessus du défaut. Dans ce type de mémoires, il est également possible de coder deux bits (soit quatre états), grâce au caractère localisé de la charge stockée. Deux types principaux de mémoires à sites de stockage discrets peuvent être cités ; les mémoires à nitrure, de type SONOS (Silicon Oxide Nitride Oxide Silicon) et les mémoires à nanocristaux de silicium.

Les mémoires à nanocristaux présentent une robustesse accrue aux défauts dans l'oxyde tunnel et permettent donc de réduire l'épaisseur (jusqu'à 5 nm) de ce dernier et ainsi de diminuer les tensions d'écriture et d'effacement et les temps de programmation.

Cependant les mémoires à nanocristaux ont aussi leurs limitations qui sont en particulier le faible couplage capacitif entre la grille de contrôle et la grille flottante. Ce qui impose de maintenir les tensions de programmation à un niveau élevé, et diminue en partie les bénéfices liés à la réduction de l'oxyde tunnel. De plus les décalages de tension de seuil obtenus sont assez faibles, à cause du faible taux de couverture de la surface active par les nanocristaux de silicium (entre 5x10¹¹ et 10¹² cm⁻²). Enfin la dispersion en taille des nanocristaux et en densité entraîne une dispersion des caractéristiques de la mémoire. D'autres types de mémoires non volatiles qui ne sont pas à base de matériaux semiconducteurs font également objet d'études avancées. Parmi les voies les plus prometteuses, on peut citer les mémoires ferromagnétiques (FeRAM), les mémoires magnétiques (MRAM) et les mémoires à changement de phase (PCRAM). Ces différentes approches ont également leurs avantages (rapidité des temps d'écriture/lecture, excellente endurance,...) et leurs inconvénients (réduction des dimensions, maturité, coût, ...*)*.

De nouvelles approches, faisant appel à la chimie, commencent à faire l'objet de recherches avancées. Ce sont les mémoires moléculaires. Elles présentent les potentialités pour aller au delà des limites des mémoires à semi-conducteurs. Les molécules de synthèse offrent en effet de nombreux avantages par rapport aux semi-conducteurs classiques : assemblage tridimensionnel, matériaux de synthèse permettant d'obtenir des propriétés sur mesure, miniaturisation approchant celle des structures biologiques, possibilités d'interface avec des systèmes vivants et surtout faible coût de fabrication. L'hybridation de ces molécules avec les systèmes CMOS actuels sera vraisemblablement la première phase de développement industriel.

Dans ces dispositifs, la charge est stockée au niveau d'un ou plusieurs atomes métalliques, ce ou ces derniers étant complexés par des molécules organiques. Afin de pouvoir stocker une charge, ces atomes métalliques doivent avoir des propriétés redox. La molécule présente alors au moins deux états de charges, un de ces états étant l'état "effacé", et l'autre, l'état "écrit". Le passage d'un état à un autre se fait par transfert de charge via un mécanisme de réaction d'oxydoréduction, en appliquant à la molécule une certaine tension de polarisation.

L'utilisation de composés organiques ayant des propriétés redox telles que les métallocènes et les métalloporphyrines a été proposée comme système à stockage de charges dans un transistor à effet de champ. Les molécules organiques complexées sont fixées de manière covalente soit directement sur le canal du transistor ou sur l'oxyde tunnel surmontant ce canal. L'accrochage de molécules organiques sur un substrat inorganique, tel que le silicium, demande une fonctionnalisation de celles-ci et/ou un traitement de surface du substrat afin de pouvoir former la liaison covalente.

Ces mémoires moléculaires à stockage de charge présentent des avantages, tels que la réduction des dimensions et l'utilisation de tensions d'alimentations faibles. En effet les potentiels redox standards de ces molécules varient en moyenne de + 2V à - 2V. D'autre part, l'utilisation de molécules possédant plus de deux états redox permettrait la mise au point d'une mémoire multi-bits.

Le problème à résoudre était de remédier aux inconvénients précités et de créer un dispositif ayant des propriétés améliorées, c'est-à-dire surtout un dispositif avec une excellente stabilité et une bonne sensibilité de détection par rapport aux dispositifs de l'art antérieur. Un autre aspect du problème à résoudre était d'offrir la possibilité de fabriquer à moindre coût, en un nombre limité d'étapes, de manière contrôlée et reproductible les dispositifs selon l'invention.

Ce problème est donc résolu par un dispositif nanostructuré selon la revendication 1.

Le dispositif selon l'invention permet l'augmentation de la surface spécifique du support solide, obtenue par la fabrication d'îlots de silicium de dimension nanométrique, avec une forte densité et une répartition spatiale plus ou moins uniforme. La modification de la géométrie du substrat, c'est-à-dire le passage d'une géométrie de deux à trois dimensions, permet d'augmenter de façon significative la surface spécifique du support. L'utilisation d'un support solide comportant des nanostructures permet donc l'accroissement de surface disponible par rapport à une surface plane, sans pour autant augmenter les dimensions du support. Les sites nanostructurés sont arrangés de manière régulière ou irrégulière en des formes géométriques qui peuvent être sélectionnées selon l'application visée. Des exemples des formes géométriques de l'arrangement des sites non limitatifs sont des cercles, carrés, lignes croisantes etc., qui sont principalement connus dans l'art.

Les surfaces des sites nanostructurés tridimensionnels sont pourvues d'une couche d'accrochage qui, dans une réalisation particulière, est poreuse. La couche d'accrochage est réalisée par une modification chimique. La modification chimique peut aussi être, par exemple, la transformation en 2 étapes d'une fonction époxyde en fonction aldéhyde pour y greffer des molécules munies de groupements de type amine. De même une fonction ester peut être transformée en fonction acide pour conduire à des surfaces hydrophiles et également réactives vis-à-vis des fonctions NH₂. Le dispositif de la présente invention comprend en outre une fonctionnalisation successive.

La surface de la couche d'accrochage est pourvue des fonctions chimiques de couplage pour permettre le greffage de molécules organiques.

Le substrat est en un matériau comprenant du silicium, germanium, quartz et mélanges dedites matériaux.

La couche intermédiaire est en matériau diélectrique ou isolant. Le matériau diélectrique est choisi parmi les oxydes, nitrures, oxydonitrures, carbures, oxydocarbures et silicates de Si, Al, Hf, Zr, TI.

Les sites nanostructurés tridimensionnels sont séparés les uns des autres et soient des grains d'une forme hémisphérique ou sphérique. Les grains ont un diamètre entre 2 et 150 nm et la densité des grains sur la couche Intermédiaire est comprise dans la plage entre 10⁶ et 10¹² cm⁻² de préférence entre 10⁹ et 10¹² cm⁻². Il est également possible que les grains soient poreux. Pour augmenter la sensibilité d'un dispositif selon l'invention, le rapport de ces deux paramètres doit être optimisé : On a observé qu'une densité des grains trop élevée conduira à la coalescence des grains, perdant l'avantage de l'accroissement de surfaces spécifiques. Donc, des densités de 5x10¹¹ à 2x10¹² .cm⁻² et des diamètres (tailles) de grain de 5 à 10 nm ou des densités de 10⁸ à 10¹⁰ cm⁻² et des tailles de 80 à 100 nm sont préférés.

Les grains utilisés dans le cadre de la présente invention sont en matériaux semi-conducteurs. Les matériaux conducteurs ou semiconducteurs sont sélectionnés parmi ceux du groupe comprenant le silicium, le carbone cristallin ou amorphe, éventuellement dopé n ou p et les métaux, par exemple nickel, platine, tungstène. Le métal préféré est nickel. Les matériaux préférables sont silicium, germanium, carbone sous forme cristalline ou amorphe, dope (graphite, nanotubes e carbone, diamant mono ou polycristallin) ou un allliage semiconducteur-metal, par exemple du siliciure de métal.

Dans le cas où les grains sont en carbone et que la couche intermédiaire est en oxyde, celle-ci sera réalisée par dépôt.

Parmi les grains pouvant être utilisés selon l'invention, trois types de grains (ou sites nanostructurés) sont spécialement préférés, dont deux sont issues de la croissance de silicium polycristallin rugueux et la troisième basée sur l'élaboration de nanocristaux de silicium. Les nanostructurés de silicium sont réalisées sur un diélectrique, en particulier sur un oxyde de silicium, par des procédés de dépôt chimique en phase vapeur essentiellement connus dans l'art.

Les espèces chimiques sont choisies parmi les acides nucléiques, les peptides, les protéines, les enzymes, les anticorps, les lipides, leurs partenaires biologiques, les composés ayant des propriétés redox comme les métallocènes, les métalloporphyrines, les polyoxométallates.

L'Invention s'applique donc aussi bien au domaine de la biologie, en particulier pour l'Immobilisation de macromolécules biologiques telles que des acides nucléiques, des lipides, des protéines (peptides, enzymes, anticorps,..) ou leurs partenaires moléculaires, permettant la réalisation de biopuces sur la base d'un dispositif selon l'Invention.

Le disosotif nanostructuré selon l'invention est obtenus par un procédé qui est encore un autre aspect de la présente invention.

Ce procédé de fabrication comprend les étapes successives selon la revendication 14.

Ce procédé selon invention offre une possibilité facile pour la fabrication des dispositifs selon l'invention. Plus spécialement, il offre la possibilité de fabriquer des biopuces avec des sites réactifs qui présentent une surface spécifique augmentée.

La couche d'accrochage est déposée sur chaque site nanostructuré pour faciliter l'étape d'accrochage e) des espèces chimiques. Cette couche d'accrochage est activée par un traitement avec un composé ayant des groupes fonctionnels aptes à fixer des espèces chimiques par une liaison chimique, de préférence par une liaison covalente. Dans un autre mode de réalisation de la présente Invention, cette liaison peut être également ionique ou autre.

Pour des applications de biopuces, les espèces chimiques à accrocher dans l'étape e) sont choisies parmi les acides nucléiques, les peptides, les protéines, les enzymes, les anticorps, les lipides, leurs partenaires biologiques, les composés ayant des propriétés redox comme les metallocènes, les metalloporphyrines, les polyoxometallates.

L'invention s'applique également au domaine de la microélectronique notamment pour la réalisation de mémoires à stockage de charges, les molécules organiques greffées sur les nanostructures de l'invention étant alors aptes à stocker ou à favoriser le stockage de charges.

Dans un mode de réalisation particulière, les espèces chimiques sont couvertes d'une couche diélectrique. En plus, une couche en un matériau conducteur est située au dessus de la couche diélectrique. Ceci permet la réalisation des mémoires électroniques.

Le problème de l'invention est également résolu par une mémoire électronique comprenant une pluralité des dispositifs selon l'invention qui sont interconnectés.

La pluralité des dispositifs forme une grille de commande et le mémoire électronique selon invention comprend une source et un drain ainsi qu'une couche d'enrobage qui est en matériau conducteur électronique ou en matériau isolant électrique.

Un autre troisième aspect du problème à résoudre était un procédé de fabrication d'une mémoire électronique selon l'invention, le procédé comportant les étapes successives selon les revendications 17 à 27.
e) accrocher des espèces chimiques sur la surface des sites nanostructurés
f) appliquer une couche d'un diélectrique de contrôle sur les espèces chimiques accrochées.
g) déposer une couche en un matériau conducteur sur la couche du diélectrique de contrôle

De préférence, le procédé selon l'invention est caractérisé en ce que l'étape b) s'effectuera par gravure successive d'une partie des couches de la grille sacrificielle.

La formation de la couche d'isolant s'effectuera préférentiellement par oxydation du substrat. Le dépôt des sites nanostructurés s'effectuera par une méthode choisie parmi CVD, LPCVD et inoculation/recuisson.

Une couche d'accrochage est déposée sur chaque site nanostructuré qui est de préférence un grain comme mentionné ci-dessus. La couche d'accrochage est activée par un traitement avec un composé ayant des groupes fonctionnels aptes à fixer des espèces chimiques par une liaison chimique, de préférence par une liaison covalente. Dans d'autres modes de réalisation une autre liaison, par exemple ionique ou tout autre type, peut être envisagée.

Dans un mode opératoire préféré, les espèces chimiques sont des molécules ayant au moins deux états d'oxydation stables comme les polyoxométallafes, les métallocenes, les métalloporphyrines, etc.

La couche d'un diélectrique de contrôle est formée par dépôt à basse température ou par addition d'un gel d'électrolyte.

### Brève description des dessins

D'autres caractéristiques et avantages de l'invention ressortiront de la description qui va suivre, en référence aux figures des dessins annexés. Les exemples de réalisation décrits avec référence aux dessins ci-annexés ne sont nullement limitatifs.
La figure 1 illustre le principe d'un dispositif selon l'invention.
La figure 2 illustre un procédé pour la préparation d'un dispositif selon la présente invention.
La figure 3 illustre la fonctionnalisation des sites nanostructurés dans un procédé pour la préparation d'un dispositif selon l'invention suivi de l'immobilisation des sondes biologiques.
La figure 4 illustre le procédé selon l'invention pour la fabrication d'un mémoire électronique selon l'invention.
La figure 5 illustre la sensibilité de détection d'un dispositif selon l'invention par rapport à un dispositif selon l'art antérieur.

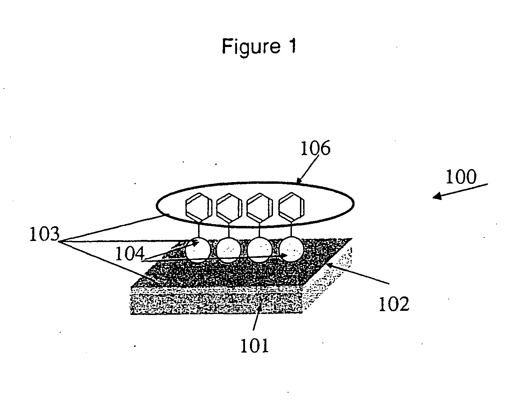

Figure 1 illustre le principe d'un support nanostructuré 100 avec un substrat 101, une couche diélectrique 102, des sites nanostructurés 103 et des espèces chimiques 104, notamment des molécules organiques accrochés sur les sites nanostructurés. Les matériaux des couches 101, 102, des sites nanostructurés 103 et la nature des espèces chimiques sont décrits ci-dessus.

Le dispositif de la présente invention pourrait être utilisée pour la préparation de capteurs chimiques et biologiques, dans le cadre de la réalisation de puces à ADN, à protéines, à sucres, à peptides, à petites molécules organiques à visée thérapeutique, et aussi pour la préparation de microsystèmes fluidiques nécessitant une fonctionnalisation de leurs parois.

En outre, le procédé de la présente invention pourrait être utilisé pour la préparation de tout dispositif de microélectronique.

### Exemple 1 : Formation du Si-polycristallin rugueux par dépôt LPCVD

Le dépôt des grains (sites nanostructurés) en couche rugueuse de silicium peut se faire directement par dépôt en phase vapeur à basse pression. Dans des conditions optimales de pression et de température, la croissance des grains se fait directement sur l'oxyde de silicium par apport permanent de silane. Les atomes de silicium sont adsorbes à la surface et leur migration successive permet d'augmenter la taille des grains.

On a utilisé un réacteur de type LPCVD (Low Pressure Chemical Vapor Decomposition) vertical. On a effectué des dépôts sur un substrat de silicium cristallin recouvert d'un oxyde thermique d'une épaisseur d'environ 1000 Ä. Pour chaque dépôt de vingt minutes, la température variait de 570 à 580°C et la pression de silane de 0,19 à 0,21 torr.

Le point de fonctionnement donnant l'accroissement de surface le plus important se situe pour une température de 580°C et une pression en silane de 200 mtorr. La surface initiale a été augmentée de 43,7%, la rugosité rms est de 16 nm et la variation de hauteur maximale entre deux points est de 106 nm.

Lors du dépôt, les atomes de silicium sont adsorbés soit directement sur le germe qui présente un site privilégie pour la nucléation, soit l'atome adsorbé migre sur la surface de SiO₂. La migration de surface a lieu jusqu'à rencontrer un germe ou un atome déjà fixé sur le germe. Ces deux phénomènes augmentent la taille du noyau progressivement, jusqu'à former un grain. On obtient un système de grains de forme hémisphérique fixés sur la couche de SiO₂. La couche n'est donc pas forcément continue. La surface de SiO₂ peut être visible entre deux grains.

Un autre procédé a été utilisé dans le contexte de l'invention pour former le film de polysilicium rugueux par décomposition thermique du silane pur dans un four vertical LPCVD puis recuit. Avant le dépôt, la pression dans l'enceinte du four est réduite à 1 mtorr. Les couches sont déposées sur substrat de silicium, oxydé thermiquement. Les conditions à rassembler lors du dépôt afin d'atteindre l'accroissement le plus important sont le couple de pression et de température (0,2 torr, 567-570°C). Le dépôt est suivi d'un recuit de 20 min in situ à une température de 570°C.

Le changement permettant de passer de la morphologie de surface d'un film de polysilicium lisse à celui d'un film le polysilicium rugueux se passe pour une variation de température lors du dépôt de quelques degrés. Ce passage d'un état de surface à l'autre est abrupt. Par contre, le changement permettant de passer de la morphologie rugueuse à celui d'un film lisse est plutôt progressif.

Pour une pression fixée (0.2 torr), l'élévation de température entraînait une augmentation rapide du nombre de grains. A basse température (550-560°C), on observe une surface amorphe et lisse avec quelques grains hémisphériques éparpillés, puis le nombre de grains augmente jusqu'à ce qu'ils adoptent une forme plus cylindrique, bien séparés les uns des autres de telle façon que le film déposé devienne discontinu (565-570°C). Pour des températures plus élevées (>575°C), on obtient un film lisse, continu et polycristallin.

L'épaisseur de la couche déposée joue un rôle important dans la morphologie de surface. A 570°C, l'épaisseur de la couche augmentant (de 60 nm à 300 nm), la densité des grains diminue et la taille de grains augmente.

Pour une température fixée (570°C), l'élévation de la pression entraîne une diminution rapide de la densité des grains. La densité est pratiquement nulle à 1 torr.

L'observation TEM permet de détecter la présence de particules cristallines dans la couche de silicium amorphe déposée à la température de transition. Ceci permet de voir aussi que le polysilicium rugueux est caractérisé par une croissance des grains de direction privilégiée <311>. La croissance du polysilicium rugueux est indépendante du substrat (SiO₂, Si₃N₄, Si). Quand le temps de recuit augmente, la taille des grains (sites nanostructurés) augmente mais la densité reste constante.

### Exemple 2 : Croissance sur silicium amorphe

Une autre méthode également applicable dans le cadre de la présente invention permet de contrôler la densité et les caractéristiques des grains. Ce procédé se déroule en trois étapes in situ. La première étape est un dépôt de silicium amorphe dopé élaboré à 540°C. La seconde, appelée « seeding » (nucléation), s'effectue à faible pression de silane (5 · 10⁻⁵ torr), à 560°C pendant 5 min. Elle permet de générer des nuclei à la surface de la couche amorphe. Ce « seeding » est suivi d'un recuit isotherme (600°C) à basse pression (3. 10⁻⁸ torr).

La présence d'oxyde natif, d'impuretés carbonées et la haute concentration de dopants dans la couche de silicium amorphe empêchent la migration de surface et donc la croissance des grains. Les effets de la pression du système et de la pression partielle du silane ne sont pas aussi importants que ceux de la température.

Les températures de dépôt doivent être les plus basses possibles car des phénomènes de cristallisation de la couche empêchent la formation de grains sur certains endroits de la plaque. La densité de nucléation augmente avec le temps de seeding.

L'étape de recuit n'intervient pas sur la densité des grains fixée lors de l'étape du seeding, mais par contre permet de définir leur taille.

La concentration de dopants est critique pour la transformation du HSG. En effet, le phosphore en surface agit comme site de nucléation et empêche la mobilité de surface du silicium atomique pendant le recuit. C'est la raison pour laquelle la couche dopée doit être couverte par une couche de silicium amorphe intrinsèque.

Ces deux voies permettent la formation de silicium polycristallin rugueux. Les sites nanostructurés obtenues sont des surfaces dites à « gros » grains (100-150 nm de diamètre), réalisées dans un four type industriel.

### Exemple 3 : Nanocristaux de silicium

Des îlots de silicium cristallins de taille nanométrique se forment par CVD pendant les premiers instants de croissance d'un film de polysilicium sur SiO₂ par CVD en chimie silane. Les températures de dépôt, proches de 600°C, sont identiques à celles utilisées pour le dépôts de silicium polycristallin. La densité et la taille des nc-Si peuvent être contrôlées par les conditions de dépôts : pression et température mais aussi par les propriétés chimiques du substrats.

Les nanodots (sites nanostructurés au sens de l'invention) sont élaborés par CVD (Chemical Vapor Déposition) sur un diélectrique en contrôlant les premiers stages de la croissance des films de silicium. La croissance est faite à partir de précurseur silane, disilane ou dichlorosilane. Le précurseur est dilué dans gaz vecteur tels que l'hydrogène ou l'hélium. La pression totale est de 20 torrs. Les dépôts se font entre 400 et 700°C avec une pression partielle comprise entre quelques milli torrs et quelques centaines de milli torrs.

La densité des nanodots peut être ajustée entre 10⁹ et quelques 10¹² cm² et la taille entre 2 et 20 nm.

Pour améliorer la dispersion en taille des nc-Si, il est nécessaire de séparer la nucléation et la croissance des nc-Si. Pour y parvenir, un procédé CVD en deux étapes est utilisé.

Dans la première étape, des nucléi de silicium (sites nanostructurés) se forment à la surface du substrat par CVD en chimie silane.

Dans la deuxième étape, le dichlorosilane (DCS) comme précurseur est utilisé car il permet une croissance sélective du silicium sur les nuclei formés durant l'étape de nucléation, sans formation de nouveaux nc-Si.

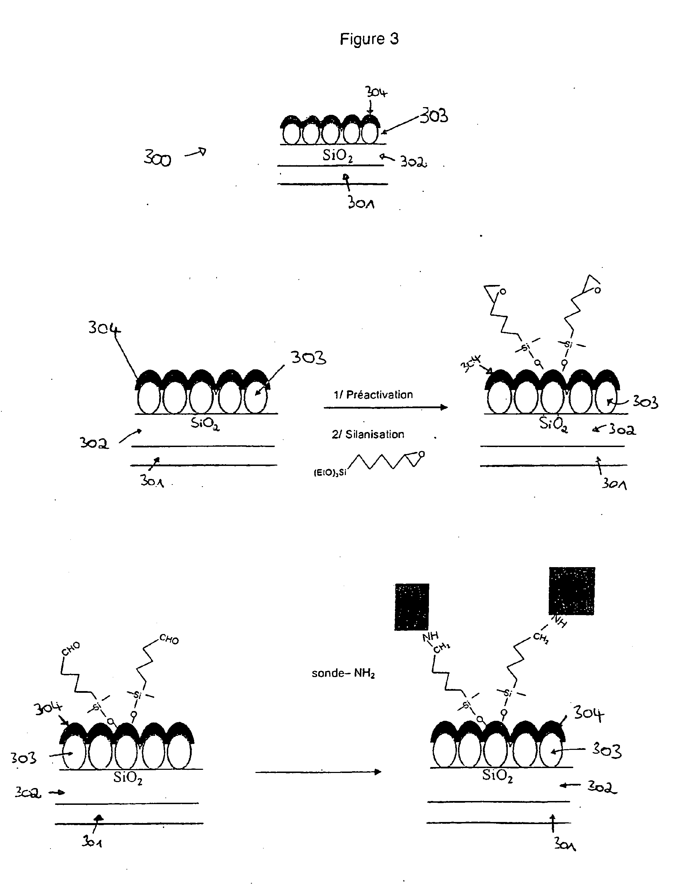

### Exemple 4 : Préparation d'une biopuce avec un dispositif selon l'invention (figure 3)

A titre d'exemple, l'étape de formation de la couche intermédiaire 304 sur des grains de silicium 303 est réalisée par oxydation thermique du silicium, comportant des liaisons Si-O-Si aptes à fixer une couche de fonctionnalisation. L'étape d'oxydation peut être réalisée en utilisant un mélange O₂/ H₂O ou O₂/HCI en atmosphère contrôlée, permettant d'obtenir des couches d'oxyde de silicium amorphe d'épaisseur variable. Le dispositif 300 obtenu est représenté dans la figure 3a. Le dispositif 300 comprend également un support 301 en silicium et une couche intermédiaire 302 en SiO₂. Les grains de Si 300 sont appliqués sur la couche 302 comme décrit ci-dessus.

L'étape de préactivation est une étape de nettoyage et de réhydratation de la surface dudit support. Elle permet la création de groupement silanols Si-OH à la périphérie des nanostructures 303 à partir des ponts siloxanes Si-O-Si, nécessaire à la fixation par liaison covalente de la couche de fonctionnalisation 304 supplémentaire. Cette étape est réalisée en milieu basique / acide et/ou oxydant ou par plasma d'O₂.

L'étape de fonctionnalisation de la surface dudit support nanostructuré est realisé par silanisation au moyen d'un réactif de silanisation comportant des fonctions aptes à fixer directement ou indirectement par des transformations chimiques successives les molécules d'intérêts, telles que par exemple des groupements époxyde, aldéhyde.

A titre d'exemple, l'étape de silanisation peut être réalisée en utilisant un réactif de silanisation possédant un groupement fonctionnel époxyde tel que le 5,6-epoxyhexyltriethoxysilane le 3-glycidoxypropyltrimethoxysilane ou bien un reactif de silanisation amine tel que le 3-aminopropyltriethoxysilane.

Selon l'organosilane utilisé pour l'étape de silanisation, il est parfois nécessaire d'effectuer d'autres transformations chimiques afin que les supports nanostructurés fonctionnalisés ainsi obtenus puissent ensuite être stockés et/ou directement utilisés pour l'immobilisation et/ou la synthèse in situ de molécule, en particulier de molécule biologiques. Suite à la fonctionnalisation citée en exemple ci-dessus, différentes étapes permettent de transformer la fonction époxyde en aldéhyde, ce groupement chimique étant apte à permettre le greffage covalent de la sonde biologique modifiée par une fonction -NH₂.

Les nanostructures ainsi fonctionnalisées peuvent être utilisées en tant qu'outil de diagnostic miniaturisé, en fonction de la nature des molécules d'intérêts fixées, comme puce à ADN par exemple pour réaliser des réactions d'hybridations avec des cibles complémentaires, comme puces à peptides, à polypeptides ou à protéines, par exemple pour la détection de réponse type antigène-anticorps. La détection de la reconnaissance biologique peut être effectuée par l'utilisation de réactifs marqués, fluorescents, radioactifs ou marqués chimiquement ou par tout autre type de mode de détection.

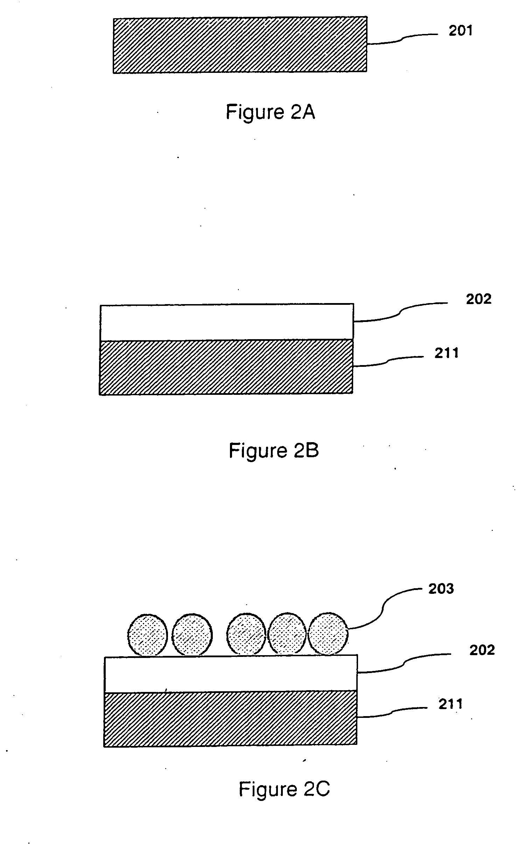

La figure 2A illustre un procédé de préparation d'un biopuce :

Un substrat 201, par exemple de silicium (100), germanium ou quartz, est recouvert (2B) d'un diélectrique 202 réalisé par exemple par oxydation du dit ou déposé directement sur le substrat 201 ou déposé sur le substrat 201 par des méthodes CVD ou PVD. Le substrat 201 peut comporter des zones dites patternées ou actives au sens utilisé par la présente invention. L'épaisseur de la couche diélectrique 202 est typiquement d'environ 100 nm.

Dans le cas d'un substrat de silicium le diélectrique peut être élaboré par oxydation thermique du substrat 201 en utilisant les procédés d'oxydation standards de la microélectronique. Des conditions typiques sont par exemple 800-1000°C en ambiance humide ou sèche. Ces procédés d'oxydation peuvent se dérouler soit en ambiance humide dilution d'eau dans un gaz vecteur inerte tel que l'azote, soit en atmosphère sèche telles que l'oxygène ou l'oxygène + chlorure d'hydrogène ou DCE, ou en présence de vapeur d'eau ou toutes autre atmosphères oxydantes utilisées dans le domaine de la microélectronique. Lorsque le diélectrique 202 est déposé, ce dernier peut être par exemple un oxyde de silicium ou un nitrure de silicium ou bien un diélectrique à forte constante diélectrique tel que par exemple un oxyde ou un silicate d'un matériau métallique, comme par exemple HfO2, HfSiOx, Al₂O₃, ZrO₂.......Pour élaborer ces diélectriques on utilise les procédés et les équipements du secteur de la microélectronique. L'épaisseur du diélectrique peut varier entre 1.2 et quelques 100 de nanomètres en fonction des besoins.

Les sites nanostructurés 203 (les « grains ») sont ensuite élaborées. Lorsque ces dernières sont en silicium, elles sont fabriquées au moyen des technologies décrites dans l'état de l'art. Les techniques de dépôt CVD du silicium ou LPCVD sont utilisées. Pour cela, un précurseur du silicium, par exemple le silane, dichlorosilane, disilane, est usité au cours d'un procédé en un ou plusieurs étapes, par exemple entre 500°C et 700°C. La densité des sites nanostructurés est comprise entre 10⁹ et 10¹⁰ cm⁻².

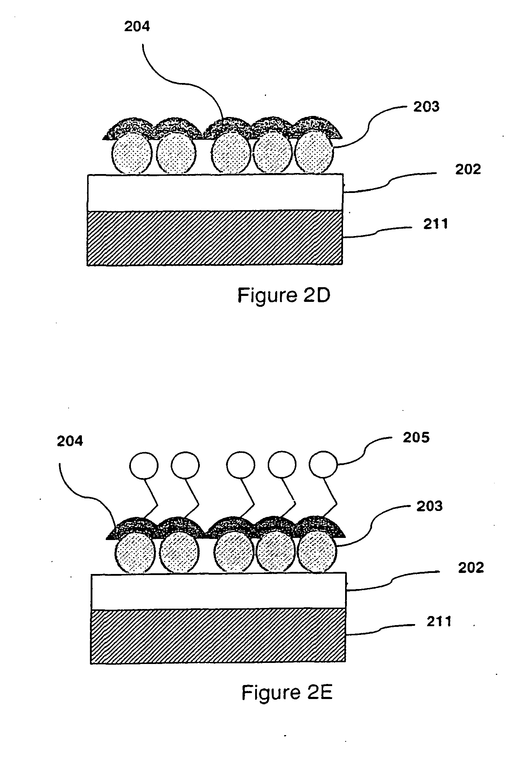

La figure 2D décrit la formation d'une couche intermédiaire d'accroche 204. Cette couche d'accroche intermédiaire 204 est obtenue à la surface des sites nanostructurés 203 par divers procédés chimiques, par exemple, par dépôt, oxydation, traitement chimique et/ou électrochimique. Préférentiellement, elle est réalisée par oxydation thermique des sites nanostructurés 203 entre 600°C et 1000°C en ambiance oxydante humide ou sèche. L'épaisseur de cette couche 205 est de l'ordre de 50 nm.

Cette couche intermédiaire, tout ou partie en un matériau doit être apte à fournir en périphérie des groupements fonctionnels aptes à permettre la fixation d'une autre couche d'accroche et/ou la fixation ou la synthèse in situ de molécules d'intérêts, et apte à permettre de limiter le couplage du rayonnement lumineux susceptible d'être émis par la biopuce suite à une excitation par un dispositif de lecture avec ledit support, dans le cas particulier de la réalisation de biopuces à détection optique. L'épaisseur de cette couche d'accroche est optimisée selon l'application visée.

Avant greffage des molécules d'intérêts 205 (figures 2E) telles que par exemple des molécules d'acide nucléique (oligonucléotides, produit PCR...), des lipides, des protéines (peptides, enzymes, anticorps...) ou leurs partenaires biologiques, il y a préactivation éventuelle des fonctions de la couche intermédiaire du support pour obtenir une surface fonctionnalisée activée, et formation d'une liaison covalente entre ladite couche de fonctionnalisation et lesdites couches intermédiaires et éventuellement activées, pour obtenir un support solide comportant des nanostructures fonctionnalisées. La couche intermédiaire 204 est préactivée par un plasma d'oxygène (600 W, 02 = 23 sccm). La préactivation permet la création de groupement silanols Si-OH à la périphérie des nanostructures à partir des ponts siloxanes Si-O-Si, nécessaire à la fixation par liaison covalente de la couche de fonctionnalisation supplémentaire.

Après préactivation, un traitement de la couche intermédiaire 204 est réalisé par silanisation au moyen d'un réactif de silanisation possédant un groupement fonctionnel époxyde tel que le 5,6-epoxyhexyltriéthoxysilane.

Ensuite, un traitement d'hydrolyse acide et une oxidation en phase liquide permettent de former des groupements aldéhydes permettant de greffer l'oligonucléotide sonde, modifié par une fonction amine (NH₂) (5' NH₂ : TTTTTGATAAACCCACTCTA).

Les sondes ainsi greffées sont ensuite hybridées par les cibles complémentaires (CATAGAGTGGGTTTATCCA) marquées par le fluorophore, Cy3 dont longueur d'onde d'excitation est 550 nm et qui réémet à 570 nm.

L'hybridation est ensuite contrôlée par une analyse optique au moyen d'un scanner Genetaq IV de marque Genonic Solutions de la société Perkin Elmer. La détection se fait à 543,5 nm.

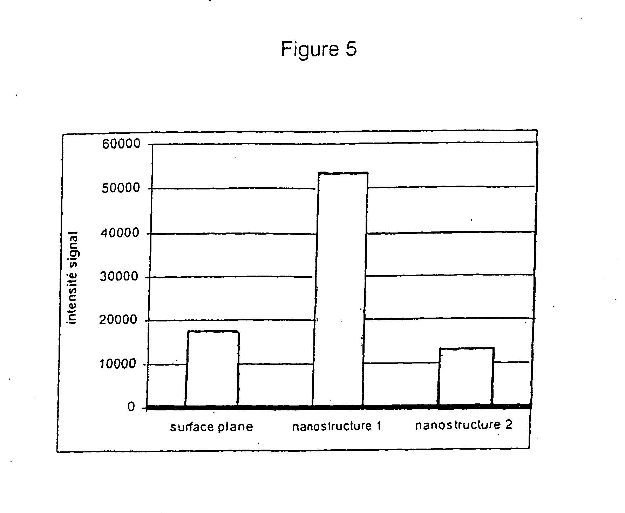

La figure 5 illustre la détection de l'hybridation des sondes précités avec les cibles mentionnés ci-dessus sur des dispositifs selon l'invention (nanostructure 1) par rapport à un dispositif préparée selon l'art antérieur (surface plane). La densité des sites nanostructurés sur le substrat était de 2 x 10⁹/cm² avec un diamètre de 33 nm des grains déposés.

L'intensité du signal utilisant un substrat plane, c'est-à-dire l'application directe des sondes sur une surface plane sans l'intermédiaire des sites nanostructurés en matériaux semiconducteurs, est beaucoup plus faible que le signal obtenu après l'hybridation sur des sites nanostructurés d'un dispositif selon l'invention. Spécifiquement, on voit dans la figure 5 qu'une densité plus faible (nanostructure 2 avec des grains plus grands) par rapport à la nanostructure 1 ne permet pas d'accroître la surface spécifique de la surface de façon significative et on voit presque aucune différence entre une surface plane et la nanostructure 2, voire même un affaiblissement de l'intensité du signal.

Pour augmenter la densité de greffage, plusieurs variantes peuvent être éventuellement envisagées. Les nanostructures 203 avant la formation éventuelle de la couche intermédiaire d'accroche peuvent être rendues poreuses par des procédés de traitements chimiques et/ou électrochimique ou tout autre traitement de porosification.

Ensuite, la formation d'une couche intermédiaire d'accroche 204 est obtenue à la surface des nanostructures 203 par divers procédés chimiques, par exemple, par dépôt, oxydation, traitement chimique et/ou électrochimique. Cette couche intermédiaire tout ou partie en un matériau doit être apte à fournir en périphérie des groupements fonctionnels aptes à permettre la fixation d'une autre couche d'accroche et/ou la fixation ou la synthèse in situ de molécules d'intérêts et apte, dans le cas d'une biopuce, à permettre de limiter le couplage du rayonnement lumineux susceptible d'être émis par la biopuce suite à une excitation par un dispositif de lecture avec ledit support, dans le cas particulier de la réalisation de biopuces à détection optique. L'épaisseur de cette couche d'accroche 204 est optimisée selon l'application visée. Dans le cas où cette couche d'accroche obtenue par oxydation, l'épaisseur de l'oxyde des nanostructures est ajustée afin de conserver l'augmentation de surface spécifique generée par les nanostructures et d'obtenir un empilement adapté à l'application visée. L'épaisseur d'oxyde peut alors varier entre quelques angströms et quelques centaines de nanomètres selon la taille des nanostructures utilisées initialement. La couche d'accroche intermédiaire 204 peut être elle même poreuse après élaboration par un procédé de la microélectronique. Il faut noter que soit les nanostructures 203 soit la couche d'accroche 204 ou bien les deux peuvent être poreux.

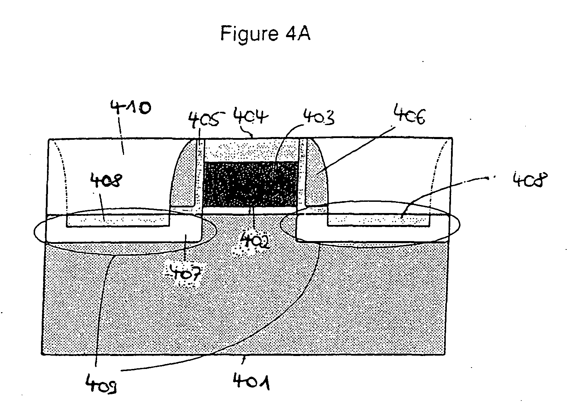

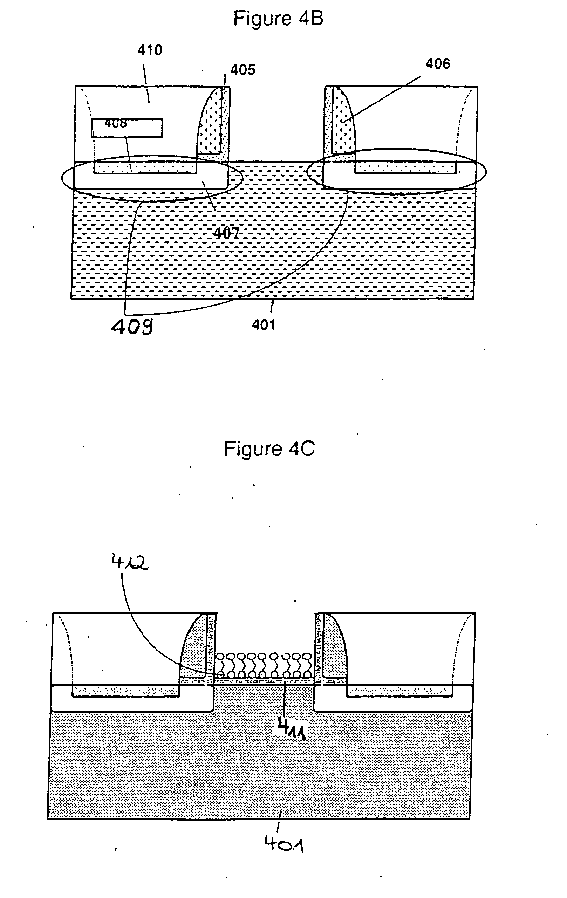

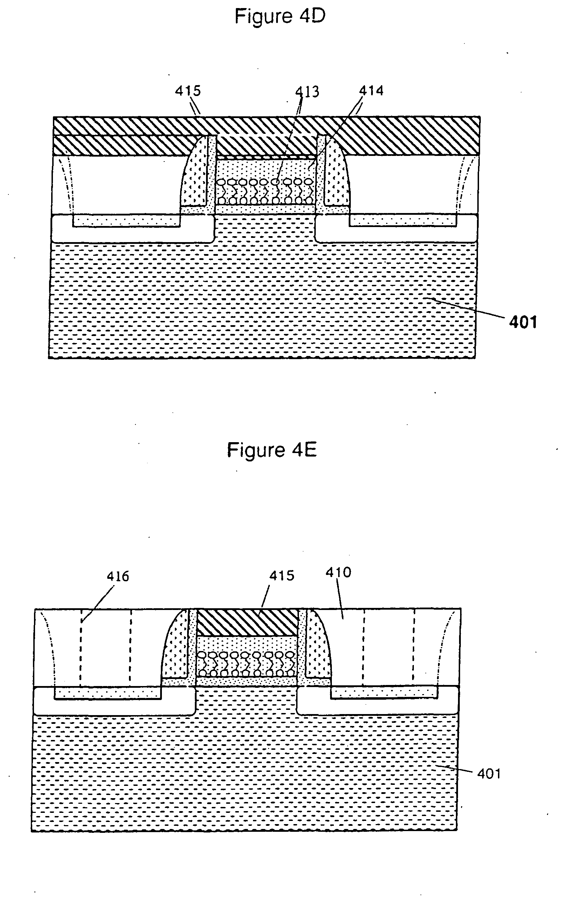

### Exemple 5 : Préparation de cellule mémoire à molécules à stockage de charges comportant des nanostructures fonctionnalisées (figure 4)

Un substrat 401, appelé base wafer, réalisé selon les procédures essentiellement comme décrites dans les références d'art antérieur est utilisé. Ce dernier est un substrat de silicium 401 les zones grille sacrificielle (402, 403, 404, 405, 406) et source 407 et drain 408 sont définies. L'épaisseur totale des couches 402, 403, 404 est par exemple de l'ordre de 100 à 500 nm et correspond à l'épaisseur totale des grilles d'un point mémoire. La couche de piédestal 402 est un oxyde réalisé par oxydation du substrat. Elle est recouverte de la couche 403 de silicium polycristallin ou amorphe, elle-même protégée par une couche de nitrure de silicium 404. La zone grille est entourée d'un espaceur en oxyde de silicium 405 et d'un espaceur en nitrure de silicium 406. Les zones source 407 et drain 408 sont constituées d'une zone dopée 409, réalisée par une ou plusieurs implantations par exemple d'ion phosphore ou arsenic avec une dose de 10¹³ à 10¹⁶ cm² à une énergie de 3 à 5 KeV, et d'une zone 410 siliciurée pour réaliser les contact électriques. Une épaisse couche 410 d'un matériau a été ensuite déposée pour enrober le device et planarisée par polissage mécano-chimique jusqu'à la grille factice (ou sacrificielle), la couche de nitrure de silicium 404 servant de couche d'arrêt. La couche 410 dans notre exemple est une couche d'oxyde. La couche 410 est soit en matériau conducteur ou isolant protège après polissage les zones sources 407 et drains 408.

Pour réaliser l'invention la première étape consiste à éliminer la grille factice délimitée par les espaceurs latéraux 405 et 406 et entourée par la couche d'enrobage 410. l'élimination de la grille se fait par gravure successive des couches 404 et 403 puis de la couche de piédestal 402 qui dans l'exemple présente est totalement éliminée (figure 5A).

La figure 4c illustre le résultat d'une première série d'opérations pour réaliser la grille définitive. Dans un premier temps une couche d'isolant 411 est réalisée au font du puit 420 réalisé à l'étape de gravure. Il s'agit par exemple d'un oxyde de silicium déposé ou obtenu par oxydation du substrat de silicium 401 sous-jacent.

Les sites nanostructurés 412 sont ensuite déposées comme décrit ci-dessus. Il s'agit par exemple de nanodots de silicium réalisé par CVD au moyen du procédé 2 étapes. Après le dépôt des nanostructures 412, les molécules organiques intéressantes 413 sont greffées après traitement au préalable des nanostructures 412 pour réaliser une couche d'accroche selon par exemple une des procédures décrites ci-dessus. Les molécules organiques 413 peuvent être par exemple des molécules ayant des propriétés redox c'est-à-dire existante sous ou moins 2 états d'oxydation comme les métallocenes, métalloporphyrines, polyoxométallates etc.

La figure 4d décrit une deuxième série d'opérations pour réaliser la grille de commande. Le diélectrique de contrôle 414, par exemple un oxyde, élaboré à basse température par les techniques d'élaboration de la microélectronique, ou un gel d'électrolyte, est déposé au dessus des molécules organiques actives. Ensuite la grille de commande 415, en un matériau conducteur tels que le silicium dopé ou un matériau métallique utilisé comme matériau de grille de commande dans le domaine de la microélectronique, comme décrit dans les références est ensuite déposé sur le diélectrique de contrôle 414. L'épaisseur de cette couche est suffisante pour entièrement remplir la partie du puit non encore occupée par les autres couches de l'empilement de grille définitif.

La figure 4e montre l'achèvement du composant mémoire. Le dépôt de la couche 415 est suivi d'une planarisation qui permet d'éliminer tous les matériaux qui dépassent au dessus de la couche d'enrobage 410 pour remettre à nu sa face superficielle. Cette operation termine le procédé de fabrication de la mémoire proprement dite. Il peut toutefois être complète par l'interconnexion du composant mémoire avec d'autres composants realisés sur le même substrat ou non. La réalisation de ces interconnections sortent du cadre strict de l'exemple de réalisation de l'invention. Elles ne sont pas décrites ici. Seuls des traits discontinus 416 indiquent la position des passages de prise de contact qu'il est possible de réaliser dans la couche 410 pour relier la source et le drain à des lignes d'interconnexion non représentées.

## Revendications

1. Dispositif nanostructuré (100, 300) comprenant un substrat (101, 201, 301, 401) en un matériau comprenant du silicium, germanium, quartz et mélanges de lesdites matériaux, une couche intermédiaire (102, 202, 302, 411) en matériau diélectrique sur une surface du substrat, une zone de ladite couche intermédiaire comportant une pluralité de sites nanostructurés tridimensionnels (104, 203, 303, 412) séparés les uns des autres et en un matériau semi-conducteur **caractérisé en ce que** les sites (104, 203, 303, 412) ont été réalisés directement sur la couche intermédiaire, sous forme de grains, d'une forme hémisphérique ou sphérique, les grains ayant un diamètre compris entre 2 et 150 nm et leur densité sur la couche intermédiaire est comprise dans la plage entre 10⁶ et 10¹² cm⁻² les surfaces des sites sont pourvues d'une couche d'accrochage (204, 304), la surface de la couche d'accrochage (204, 304) étant activée par un traitement avec un composé ayant des fonctions chimiques de couplage aptes a fixer aux sites nanostructurés des espèces chimiques (106, 205, 413) par des liaisons chimiques.

2. Dispositif selon la revendication 1, **caractérisé en ce que** les espèces chimiques sont fixés aux sites nanostructurés par des liaisons covalentes.

3. Dispositif selon la revendication 1 ou 2, **caractérisé en ce que** la couche d'accrochage est poreuse.

4. Dispositif selon l'une des revendications 1 à 3, **caractérisé en ce que** le matériau diélectrique est choisi parmi les oxydes, nitrures, oxydonitrures, carbures, oxydocarbures et silicates de Si, Al, Hf, Zr, Tl.

5. Dispositif selon la revendication 1, **caractérisé en ce que** les grains sont poreux.

6. Dispositif selon l'une des revendications précédentes, **caractérisé en ce que** les espèces chimiques sont choisies parmi les acides nucléiques, les peptides, les protéines, les enzymes, les anticorps, les lipides, leurs partenaires biologiques, les composés ayant des propriétés redox comme les métallocènes, les métalloporphyrines, les polyoxométallates.

7. Biopuce **caractérisé en ce qu'**elle comporte une pluralité des dispositifs selon l'une des revendications précédentes 1 à 6

8. Dispositif selon l'une des revendications précédentes 1 à 6, **caractérisé en ce que** les espèces chimiques sont couvertes d'une couche diélectrique.

9. Dispositif selon la revendication 8, **caractérisé en ce qu'**une couche en un matériau conducteur est situé au dessus de la couche diélectrique.

10. Mémoire électronique **caractérisé en ce qu'**il comprend une pluralité des dispositifs selon la revendication 9 qui sont interconnectés.

11. Mémoire électronique selon la revendication 10, **caractérisé en ce que** la pluralité des dispositifs forme une grille de commander (415).

12. Mémoire électronique selon la revendication 11 comprenant une source (407) et un drain (408).

13. Mémoire électronique selon la revendication 12 présentant une couche d'enrobage (410) qui est en matériau conducteur électronique ou en matériau isolant électrique.

14. Procédé de fabrication d'un dispositif nanostructuré (100, 300) selon l'une des revendications 1 à 6, **caractérisé en ce que** le procédé comporte les étapes successives suivantes :
a) se munir d'un substrat (101,201, 301, 401) en un matériau comprenant du silicium, germanium, quartz et mélanges de lesdites matériaux
b) former une couche intermédiaire (102, 202, 302, 411) en matériau diélectrique sur une surface du substrat
c) réaliser des sites nanostructurés tridimensionnels (104) séparés les uns des autres consistant
en un matériau semi-conducteur directement sur la couche intermédiaire sous forme de grains d'une forme hémisphérique ou sphérique ayant un diamètre compris entre 2 et 150 nm avec une densité comprise dans la plage entre 10⁶ et 10¹² cm⁻²
d) déposer une couche d'accrochage (204, 304) sur chaque site nanostructuré (104, 203, 303, 412)
e) activer la couche d'accrochage par un traitement avec un composé ayant des groupes fonctionnels aptes à fixe au site nanostructuré des espèces chimiques par une liaison chimique.

15. Procédé selon la revendication 14 comportant l'étape supplémentaire f) d'accrocher après l'étape e) des espèces chimiques (106, 205, 413) sur la surface des sites nanostructurés tridimensionnels.

16. Procédé selon la revendication 15, **caractérisé en ce que** les espèces chimiques sont choisies parmi les acides nucléiques, les peptides, les protéines, les enzymes, les anticorps, les lipides, leurs partenaires biologiques, les composés ayant des propriétés redox comme les métallocènes, les métalloporphyrines, les polyoxométallates.

17. Procédé selon l'une des revendications 14 à 16 pour la fabrication d'une mémoire électronique (400) selon l'une des revendications 10 à 13, **caractérisé en ce que** le substrat (401) est en silicium et comporte des zones de grille sacrificielle (402, 403, 404, 405, 406), des sources (407), des drains (408), une zone siliciurée (409) et une couche d'enrobage (410).

18. Procédé selon la revendication 17, le procédé comportant avant l'étape b) l'étape suivante :
b₁) éliminer la grille sacrificielle par la zone de grilles jusqu'à la surface du substrat (401) pour former un puits (420) de taille définie

19. Procédé selon la revendication 18, **caractérisé en ce que** les étapes b) et c) sont
b) former une couche d'isolant electrique (411) la surface du substrat (401) obtenue dans l'étape b₁
c) déposer des sites nanostructurés (412) sur la couche d'isolant (411) au fond du puits (420)

20. Procédé selon la revendication 19, **caractérisé par** les étapes additionnelles suivantes :
g) appliquer une couche d'un diélectrique de contrôle (414) sur les espèces chimiques accrochées
h) déposer une couche en un matériau conducteur (415) sur la couche du diélectrique de contrôle (414)

21. Procédé selon l'une des revendications précédentes) 18 à 20, **caractérisé en ce que** l'élimination de la grille sacrificielle par gravure successive de couches de zones de grille sacrificielle (402, 403, 404).

22. Procédé selon la revendication 19, **caractérisé en ce que** la formation de la couche d'isolant électrique (411) s'effectuera par oxydation du substrat (401).

23. Procédé selon la revendication 19 **caractérisé en ce que** le dépôt des sites nanostructurés (412) s'effectuera par une méthode choisie parmi CVD, LPCVD et inoculation/recuisson.

24. Procédé selon l'une des revendications 17 à 23 **caractérisé en ce qu'**une couche d'accrochage est déposée sur chaque site nanostructuré (412).

25. Procédé selon la revendication 24, **caractérisé en ce que** la couche d'accrochage est activée par un traitement avec un composé ayant des groupes fonctionnels aptes à fixer des espèces chimiques par une liaison covalente.

26. procédé selon l'une des revendications 17 à 25, **caractérisé en ce que** les espèces chimiques sont des molécules ayant au moins deux états d'oxydation stables.

27. Procédé selon la revendication 20 **caractérisé en ce que** la couche d'un diélectrique de contrôle (414) est formée par dépôt à basse température ou par addition d'un gel d'électrolyte.

## Claims

1. A nanostructured device (100, 300) comprising a substrate (101, 201, 301, 401) made of a material comprising silicon, germanium, quartz and mixtures of said materials, an intermediate layer (102, 202, 302, 411) made of a dielectric material on a surface of the substrate, a zone of said intermediate layer comprising a plurality of three-dimensional nanostructured sites (104, 203, 303, 412) separated one from the other and consisting of a semi-conductor material, **characterized in that** the sites (104, 203, 303, 412) have been produced directly on the intermediate layer in the form of grains having a hemispherical or spherical shape with a diameter of between 2 and 150 nm, their density on the intermediate layer being in the range between 10⁶ and 10¹² cm⁻², the site surfaces being equipped with a tie layer (204, 304), the surface of the tie layer (204, 304) being activated by treatment with a compound having chemical coupling functions capable of fixing chemical species (106, 205, 413) to the nanostructured sites chemical species via chemical bonds.

2. The device according to claim 1, **characterized in that** the chemical species are fixed to the nanostructured sites by covalent bonds.

3. The device according to claim 1 or 2, **characterized in that** the tie layer is porous.

4. The device according to one of the claims 1 to 3, **characterized in that** the dielectric material is chosen from oxides, nitrides, oxynitrides, carbides, oxycarbides and silicates of Si, Al, Hf, Zr, Tl.

5. The device according to claim 1, **characterized in that** the grains are porous.

6. The device according to one of the preceding claims, **characterized in that** the chemical species are chosen from nucleic acids, peptides, proteins, enzymes, antibodies, lipids, their biological partners, compounds having redox properties such as metallocenes, metalloporphyrins and polyoxometallates.

7. A biochip **characterized in that** it comprises a plurality of devices according to one of the preceding claims 1 to 6.

8. The device according to one of the preceding claims 1 to 6, **characterized in that** the chemical species are covered with a dielectric layer.

9. The device according to claim 8, **characterized in that** a layer made of a conductive material is located on top of the dielectric layer.

10. An electronic memory **characterized in that** it comprises a plurality of devices according to claim 9 which are interconnected.

11. The electronic memory according to claim 10, **characterized in that** the plurality of devices forms a control gate (415).

12. The electronic memory according to claim 11, comprising a source (407) and a drain (408).

13. The electronic memory according to claim 12, exhibiting an encapsulation layer (410) which is made of an electronically conductive material or an electrically insulating material.

14. A process for manufacturing a monostructured device (100, 300) according to one of the claims 1 to 6, **characterized in that** the process comprises the following successive steps:
a) providing a substrate (101, 201, 301, 401) made of a material comprising silicon, germanium, quart and mixtures of said materials;
b) forming an intermediate layer (102, 202, 302, 411) made of dielectric material on a surface of the substrate;
c) producing three-dimensional nanostructured sites (104) separated one from the other consisting of a semiconductor material directly on the intermediate layer in the form of grains having a hemispherical or spherical shape with a diameter of between 2 and 250 nm and with a density in the range between 1⁻⁶ and 10¹² cm⁻²;
d) depositing a tie layer (204, 304) on each nanostructured site (104, 203, 303, 412);
e) activating the tie layer by treatment with a compound that has functional groups capable of fixing chemical species to the nanostructured site via a chemical bond.

15. The process according to claim 14 comprising the additional step f) after step e) of tying chemical species (106, 305, 413) to the surface of the three-dimensional nanostructured sites.

16. The process according to claim 15, **characterized in that** the chemical species are chosen from nucleic acids, peptides, proteins, enzymes, antibodies, lipids, their biological partners, compounds having redox properties such as metallocenes, metalloporphyrins and polyoxometallates.

17. The process according to one of the claims 14 to 16 for manufacturing an electronic memory (400) according to one of the claims 10 to 13, **characterized in that** the substrate (401) is made of silicon and comprises sacrificial gate zones (402, 403, 404, 405, 406), sources (407), drains (408), a silicided zone (409) and an encapsulation layer (410).

18. The process according to claim 17, the process comprising, before step b), the following step:
b₁) removing the sacrificial gate by the gate zone down to the surface of the substrate (401), in order to form a well (420) of defined size.

19. The process according to claim 18, **characterized in that** the steps b) and c) are carried out by:
b) forming an electrical insulating layer (411) over the surface of the substrate (401) obtained in step b₁;
c) depositing nanostructured sites (412) over the insulating layer (411) at the bottom of the well (420).

20. The process according to claim 19, **characterized by** the following additional steps:
g) applying a layer of a control dielectric (414) over the tied chemical species
h) depositing a layer made from a conductive material (415) over the control dielectric layer (414).

21. The process according to one of the preceding claims 18 to 20, **characterized in that** the removal of the sacrificial gate is carried out by successive etching of layers of sacrificial gate zones (402, 403 and 404).

22. The process according to claim 19, **characterized in that** the formation of the dielectric insulating layer will be carried out by oxidation of the substrate (401).

23. The process according to claim 19, **characterized in that** the deposition of nanostructured sites (412) will be carried out by a method chosen from CVD, LPCVD and inoculation/annealing.

24. The process according to one of the claims 17 to 23, **characterized in that** a tie layer is deposited on each nanostructured site (412).

25. The process according to claim 24, **characterized in that** the tie layer is activated by treatment with a compound that has functional groups capable of fixing chemical species via a covalent bond

26. The process according to one of the claims 17 to 25, **characterized in that** the chemical species are molecules that have at least two stable oxidation states.

27. The process according to claim 20, **characterized in that** the layer of a control dielectric (414) is formed by low-temperature deposition or by addition of an electrolyte gel.

## Patentansprüche

1. Nanostrukturierte Vorrichtung (100, 300), umfassend ein Substrat (101, 201, 301, 401) aus einem Material, das Silicium, Quarz und Mischungen der beiden Materialien umfasst, eine Zwischenschicht (102, 202, 302, 411) aus dielektrischem Material auf einer Seite des Substrats, einen Bereich der Zwischenschicht, welcher dreidimensionale nanostrukturierte Stellen (104, 203, 303, 412) aufweist, die voneinander getrennt sind und aus einem Halbleitermaterial bestehen, **dadurch gekennzeichnet, dass** die Stellen (104, 203, 303, 412) direkt auf der Zwischenschicht hergestellt wurden, in Form von Körnern mit einer halbkugelartigen oder kugelartigen Form, wobei die Körner einen Durchmesser im Bereich von 2 und 150 nm haben und ihre Dichte auf der Zwischenschicht im Bereich von 10⁶ und 10¹² cm⁻² liegt, wobei die Oberflächen der Stellen mit einer Anhaftschicht (204, 304) versehen sind, wobei die Oberfläche der Anhaftschicht (204, 304) durch eine Behandlung mit einer Verbindung aktiviert wird, welche chemische Kupplungsfunktionen hat, die dazu befähigt sind, an nanostrukturierte Stellen von chemischen Spezies (106, 205, 413) zu binden, indem sie chemischen Bindungen eingehen.

2. Vorrichtung nach Anspruch 1, **dadurch gekennzeichnet, dass** die chemischen Spezies durch kovalente Bindungen an die nanostrukturierten Stellen gebunden sind.

3. Vorrichtung nach Anspruch 1 oder 2, **dadurch gekennzeichnet, dass** die Anhaftschicht porös ist.

4. Vorrichtung nach einem der Ansprüche 1 bis 3, **dadurch gekennzeichnet, dass** das dielektrische Material aus den Oxiden, Nitriden, Oxidonitriden, Carbiden, Oxidocarbiden und Silikaten von Si, Al, Hf, Zr, Ti ausgewählt ist.

5. Vorrichtung nach Anspruch 1, **dadurch gekennzeichnet, dass** die Körner porös sind.

6. Vorrichtung nach einem der vorhergehenden Ansprüche, **dadurch gekennzeichnet, dass** die chemischen Spezies aus den Nukleinsäuren, den Peptiden, den Proteinen, den Enzymen, den Antikörpern, den Lipiden, ihren biologischen Partnern ausgewählt sind, wobei die Verbindungen Redoxeigenschaften wie Metallocene, Metalloporphyrine, Polyoxometallate haben.

7. Biochip, der **dadurch gekennzeichnet ist, dass** er mehrere Vorrichtungen nach einem der vorhergehenden Ansprüche 1 bis 6 aufweist.

8. Vorfahren nach einem der vorhergehenden Ansprüche 1 bis 6, **dadurch gekennzeichnet, dass** die chemischen Spezies von einer dielektrischen Schicht bedeckt sind.

9. Vorrichtung nach Anspruch 8, **dadurch gekennzeichnet, dass** sich eine Schicht aus einem leitfähigen Material auf der dielektrischen Schicht befindet.

10. Elektronischer Speicher, **dadurch gekennzeichnet, dass** er mehrere Vorrichtungen nach Anspruch 9 umfasst, die miteinander verbunden sind.

11. Elektronischer Speicher nach Anspruch 10, **dadurch gekennzeichnet, dass** die mehreren Vorrichtungen ein Steuergitter (415) bilden.

12. Elektronischer Speicher nach Anspruch 11, der eine Quelle (407) und eine Senke (408) umfasst.

13. Elektronischer Speicher nach Anspruch 12, der eine Umhüllungsschicht (410) aufweist, die aus einem elektronenleitenden Material oder einem elektrisch isolierenden Material besteht.

14. Verfahren zur Herstellung einer nanostrukturierten Vorrichtung (100, 300) nach einem der Ansprüche 1 bis 6, **dadurch gekennzeichnet, dass** das Verfahren die folgenden Schritte aufweist:
a) Bereitstellen eines Substrats (101, 201, 301, 401) aus einem Material, das Silicium, Germanium, Quarz und Mischungen aus diesen Materialien umfasst
b) Bilden einer Zwischenschicht (102, 202, 302, 411) aus dielektrischem Material auf einer Oberfläche des Substrats.
c) Herstellen der dreidimensionalen nanostrukturierten Stellen (104), die voneinander getrennt sind und aus einem Halbleitermaterial bestehen, direkt auf der Zwischenschicht in Form von halbkugel- oder kugelförmigen Körnern, welche einen Durchmesser im Bereich von 2 bis 150 nm haben, mit einer Dichte im Wertebereich von 10⁶ bis 10¹² cm⁻²
d) Abscheiden einer Anhaftschicht (204, 304) auf jeder nanostrukturierte Stelle (104, 203, 303, 412)
e) Aktivieren der Anhaftschicht durch Behandlung mit einer Verbindung, die funktionelle Gruppen hat, welche dazu befähigt sind, an die nanostrukturierte Stelle von chemischen Spezies zu binden, indem sie eine chemische Bindung eingehen.

15. Verfahren nach Anspruch 14, das nach dem Schritt e) einen zusätzlichen Schritt f) zum Anhaften der chemischen Spezies (106, 205) an die Oberfläche der dreidimensionalen nanostrukturierten Stellen aufweist.

16. Verfahren nach Anspruch 15, **dadurch gekennzeichnet, dass** die chemischen Spezies aus den Nukleinsäuren, den Peptiden, den Proteinen, den Enzymen, den Antikörpern, den Lipiden, ihren biologischen Partnern ausgewählt sind, wobei die Verbindungen Redoxeigenschaften wie Metallocene, Metalloporphyrine, Polyoxometallate haben.

17. Verfahren nach einem der Ansprüche 14 bis 16 zur Herstellung eines elektronischen Speichers (400) nach einem der Ansprüche 10 bis 13, **dadurch gekennzeichnet, dass** das Substrat (401) aus Silicium ist und Opfergitterbereiche (402, 403, 404, 405, 406), Quellen (407), Senken (408), einen silicidartigen Bereich (409) und eine Umhüllungsschicht (410) aufweist.

18. Verfahren nach Anspruch 17, wobei das Verfahren vor dem Schritt b) den folgenden Schritt aufweist:
b₁) Entfernen des Opfergitters durch den Gitterbereich bis zur Oberfläche des Substrats (401), um einen Schacht (420) von bestimmter Größe zu bilden.

19. Verfahren nach Anspruch 18, **dadurch gekennzeichnet, dass** die Schritte b) und c) folgendermaßen durchgeführt werden:
b) Bilden einer elektrisch isolierenden Schicht (411) auf der Oberfläche des Substrats (401), welches in Schritt b₁ erhalten wurde
c) Abscheiden von nanostrukturierten Stellen (412) auf der Isolierschicht (411) am Boden des Schachtes (420)

20. Verfahren nach Anspruch 19, **gekennzeichnet durch** die folgenden zusätzlichen Schritte :
g) Aufbringen einer Schicht aus einer Schicht aus einem Prüfdielektrikum (414) auf die anhaftenden chemischen Spezies
h) Abscheiden einer Schicht aus einen leitfähigen Material (415) auf der Schicht des Prüfdielektrikums (414)

21. Verfahren nach einem der vorhergehenden Ansprüche 18 bis 20, **dadurch gekennzeichnet, dass** das Entfernen des Opfergitters durch aufeinanderfolgendes schichtweises Ätzen von Opfergitterbereichen (402, 403, 404) erfolgt.

22. Verfahren nach Anspruch 19, **dadurch gekennzeichnet, dass** die Bildung der elektrischen Isolierschicht (411) durch Oxidation des Substrats (401) erfolgt.

23. Verfahren nach Anspruch 19, **dadurch gekennzeichnet, dass** das Abscheiden der nanostrukturierten Stellen (412) durch ein Verfahren erfolgt, welches aus CVD, LPCVD und Beimpfen/Glühen ausgewählt ist.

24. Verfahren nach einem der Ansprüche 17 bis 23, **dadurch gekennzeichnet, dass** eine Anhaftschicht auf jeder nanostrukturierten Stelle (412) abgeschieden wird.

25. Verfahren nach Anspruch 24, **dadurch gekennzeichnet, dass** die Anhaftschicht durch eine Behandlung mit einer Verbindung aktiviert wird, die funktionelle Gruppen hat, welche dazu befähigt sind, chemische Spezies zu binden, indem sie eine kovalente Bindungen eingeht.

26. Verfahren nach einem der Ansprüche 17 bis 25, **dadurch gekennzeichnet, dass** es sich bei den chemischen Spezies um Moleküle mit mindestens zwei stabilen Oxidationszuständen handelt.

27. Verfahren nach Anspruch 20, **dadurch gekennzeichnet, dass** die Schicht aus einem Prüfdielektrikum (414) durch Abscheiden bei niedriger Temperatur oder durch Zusatz eines Elektrolytgels gebildet wird.
